# EUROPÄISCHE PATENTSCHRIFT

(11) **EP 4 472 536 B1**
(45) Veröffentlichungstag und Bekanntmachung des Hinweises auf die Patenterteilung: **18.02.2026**
(21) Anmeldenummer: 23705467.1
(22) Anmeldetag: 18.01.2023
(51) Int. Cl.: A61B 17/74, A61B 17/80

(54) **VORRICHTUNG ZUM VERSORGEN EINER FRAKTUR**
DEVICE FOR TREATING A FRACTURE
DISPOSITIF DE TRAITEMENT D'UNE FRACTURE

(30) Priorität: 01.02.2022 AT 500512022
(43) Veröffentlichungstag der Anmeldung: 11.12.2024
(73) Patentinhaber: I.T.S. GmbH, 8301 Lassnitzhöhe (AT)
(72) Erfinder: BÜHREN, Volker, 82418 Murnau (DE); PRAGER, Ronald, 24796 Bovenau (DE)
(74) Vertreter: Wirnsberger & Lerchbaum Patentanwälte OG
(86) Internationale Anmeldenummer: PCT/AT2023/060009
(87) Internationale Veröffentlichungsnummer: WO 2023/147609

(56) Entgegenhaltungen:
- WO-A1-2009/043827
- DE-A1- 102019 108 264
- JP-A- 2004 097 446
- US-A1- 2005 055 024
- US-A1- 2018 177 538

## Beschreibung

Die Erfindung betrifft eine Vorrichtung zum Versorgen einer Fraktur, insbesondere einer Fraktur eines proximalen Femurs wie beispielsweise einer Schenkelhalsfraktur, aufweisend eine Platte zur Anbringung an einem Oberschenkelknochen, eine oder mehrere Intraossärschrauben, mit welchen die Platte durch Intraossärschraubenöffnungen hindurch an einem Oberschenkelschaft fixierbar ist, eine proximal zu der zumindest einen Intraossärschraube angeordnete Koppeleinrichtung, insbesondere eine Knochenschraube, mit welcher die Platte an einer Koppeleinrichtungsöffnung durch den Oberschenkelschaft und einen Schenkelhals hindurch mit einem Oberschenkelkopf verbindbar ist, wobei die Koppeleinrichtung mit einem Spiel mit der Platte verbunden ist, sodass die Koppeleinrichtung in Lateralrichtung relativ zur Platte bewegbar ist. Eine solche Vorrichtung ist beispielsweise aus US2005/055024 A1 bekannt.

Aus dem Stand der Technik sind Vorrichtungen der eingangs genannten Art bekannt geworden, um eine Fraktur eines Schenkelhalses zu versorgen, indem die Knochenplatte einerseits mittels der Intraossärschrauben mit dem Oberschenkelschaft verbunden wird, wonach der Oberschenkelkopf durch die Koppeleinrichtung, welche üblicherweise als Knochenschraube ausgebildet ist, mit der Platte verbunden wird. Eine bewegbare Verbindung der Koppeleinrichtung mit der Platte ist dabei günstig, um einen Heilungsprozess zu fördern. So hat sich gezeigt, dass kleine Bewegungen und Kompression an einer Kontaktfläche zwischen den Teilen der Knochenfraktur, also üblicherweise an einer Kontaktfläche im Bereich des Schenkelhalses, günstig für eine Förderung des Heilungsprozesses sind, wobei allerdings ein zu starkes Ineinandergleiten der Knochenteile vermieden werden muss.

Die aus dem Stand der Technik bekannten Vorrichtungen, welche insbesondere sogenannte Barrel- und/oder Teleskopschrauben aufweisen und beispielsweise aus dem Dokument WO 2007/109302 A2 bekannt sind, haben sich jedoch als unhandlich erwiesen.

Hier setzt die Erfindung an. Aufgabe der Erfindung ist es, eine Vorrichtung der eingangs genannten Art anzugeben, welche einerseits einfach handhabbar und andererseits für einen Heilungsprozess besonders günstig ist.

Diese Aufgabe wird erfindungsgemäß durch eine Vorrichtung der eingangs genannten Art gelöst, bei welcher eine mit der Platte verbundene und an unterschiedlichen Positionen relativ zur Platte positionierbare Justiereinrichtung vorgesehen ist, mit welcher das Spiel einstellbar ist.

Aufgrund der erfindungsgemäßen Ausbildung der Vorrichtung ist ein Einsatz von Teleskopschrauben oder dergleichen somit nicht mehr erforderlich, sondern wurde im Rahmen der Erfindung erkannt, dass eine robuste Bauweise und gleichzeitig eine einfache Einstellbarkeit des Spiels auf einfache Weise durch eine entsprechende Justiereinrichtung erreicht werden können. Die Justiereinrichtung kann dabei grundsätzlich auf unterschiedlichste Weisen ausgebildet sein, um eine Größe eines Spiels zu beeinflussen, innerhalb dessen die Koppeleinrichtung relativ zur Platte bewegbar ist.

Günstig ist es, wenn die Justiereinrichtung in Lateralrichtung und Medialrichtung im Wesentlichen starr mit der Platte verbunden ist. Dies kann beispielsweise durch eine Justiereinrichtung erreicht werden, welche durch eine in die Platte einschraubbare Justierschraube gebildet ist und mittels welcher eine Bewegbarkeit der Koppeleinrichtung relativ zur Platte einstellbar ist. Es kann dann das Spiel der Koppeleinrichtung relativ zur Platte durch eine Einschraubtiefe der Justierschraube eingestellt werden.

Besonders bevorzugt ist vorgesehen, dass die Koppeleinrichtung eine erste Anschlagfläche aufweist, welche mit einer korrespondierenden Fläche an der Justiereinrichtung und/oder der Platte zusammenwirkt, sodass durch die erste Anschlagfläche eine Bewegbarkeit der Koppeleinrichtung relativ zur Platte in Medialrichtung begrenzt ist. Die Koppeleinrichtung kann somit relativ zur Platte in Medialrichtung bewegt werden, bis diese an der ersten Anschlagfläche anliegt, sodass durch die erste Anschlagfläche eine mediale Endposition der Koppeleinrichtung relativ zur Platte definiert wird.

Diese erste Anschlagfläche kann grundsätzlich in jeder beliebigen Weise ausgebildet sein, die ab einer vordefinierten Position der Koppeleinrichtung in Medialrichtung an der Justiereinrichtung und/oder der Platte anliegt und damit eine weitere Bewegung in Medialrichtung verhindert. Die Anschlagfläche kann somit sowohl normal auf eine Längsachse der Koppeleinrichtung, entlang welcher diese Koppeleinrichtung relativ zur Platte bewegbar ist, ausgerichtet sein als auch unter einem Winkel zur Längsachse, um eine besonders geringe Flächenpressung zu erreichen.

Beispielsweise kann vorgesehen sein, dass die Koppeleinrichtung lateral einen Bund aufweist, welcher im Zusammenwirken mit der Platte einen Anschlag bildet, bis zu welchem die Koppeleinrichtung medial in die Platte einführt werden kann. Die Koppeleinrichtung kann dann einfach als Zugschraube eingesetzt werden, welche den Oberschenkelkopf an die Platte heranzieht. Die Koppeleinrichtung weist dann beispielsweise an einem lateralen Ende einen Bund bzw. eine Schulter auf, welche größer als die Koppeleinrichtungsöffnung ausgebildet ist, sodass die Koppeleinrichtung in medialer Richtung nur bis zum Bund in die Platte einführbar ist.

Günstig ist es, wenn die Koppeleinrichtung eine zweite Anschlagfläche aufweist, welche in einer lateralen Endposition der Koppeleinrichtung an der Justiereinrichtung anliegt. Somit kann durch eine Positionsänderung der Justiereinrichtung relativ zur Platte auf einfache Weise ein Spiel verändert werden, zumal dadurch ein Abstand zwischen den Anschlägen verändert wird.

Die zweite Anschlagfläche kann durch nur eine einzige Fläche gebildet sein, welche in der lateralen Endposition einen Kontakt zwischen Koppeleinrichtung und Justiereinrichtung herstellt. Es kann allerdings auch vorgesehen sein, dass zweite Anschlagfläche durch mehrere Flächen gebildet ist, welche parallel wirken und somit eine Bewegbarkeit besonders effektiv begrenzen. So hat sich gezeigt, dass bei Einsatz nur einer einzigen zweiten Anschlagfläche, welche insbesondere an einem medialen Gewindeende der Justiereinrichtung anliegt, ein Risiko besteht, dass die Justiereinrichtung entlang der Koppeleinrichtung gleitet bzw. sich über das Gewinde auf die zweite Anschlagfläche an der Koppeleinrichtung aufschraubt, sodass die Bewegbarkeit weniger als intendiert begrenzt ist. Insbesondere kann vorgesehen sein, dass die zweite Anschlagfläche durch zwei oder drei parallel wirkende Anschläge bzw. Kontaktflächen gebildet ist, beispielsweise durch einen primären, einen sekundären und einen tertiären zweiten Anschlag. Dabei kann der primäre zweite Anschlag beispielsweise an einem lateralen Ende der Justiereinrichtung, der sekundäre zweite Anschlag beispielsweise mittig an der Justiereinrichtung und der tertiäre zweite Anschlag im Bereich eines medialen Endes der Justiereinrichtung an der Justiereinrichtung anliegen.

Günstig ist es, wenn die zweite Anschlagfläche zumindest teilweise durch eine laterale zweite Anschlagfläche gebildet wird, welche in der lateralen Endposition zumindest teilweise an einer Schulter oder einem Kragen der Justiereinrichtung, insbesondere an einem Justierschraubenkragen, in einem Bereich eines lateralen Endes der Justiereinrichtung anliegt. Der bevorzugt als Justierschraubenkragen ausgebildete Kragen kann an einem lateralen Ende der Justierschraube angeordnet sein und einen größeren Durchmesser als die übrige Justiereinrichtung aufweisen, sodass die Koppeleinrichtung an der lateralen Endposition an der Justiereinrichtung anliegt, und zwar an einem lateralen Ende der Justiereinrichtung. In dem Fall kann die zweite Anschlagfläche an der Koppeleinrichtung an einer endseitigen, lateralen umlaufenden Vertiefung angeordnet sein, um einen entsprechenden Formschluss mit dem Kragen an der Justiereinrichtung zu erreichen.

Bevorzugt ist vorgesehen, dass die zweite Anschlagfläche zumindest teilweise durch eine mediale zweite Anschlagfläche an der Koppeleinrichtung gebildet wird, welche in der lateralen Endposition zumindest teilweise an einem Gewinde der Justiereinrichtung anliegt. Insbesondere ein mediales Ende des Gewindes an der Justiereinrichtung kann somit als Anschlag für eine korrespondierende Fläche der Koppeleinrichtung dienen, um eine Bewegbarkeit der Koppeleinrichtung in Lateralrichtung zu begrenzen.

Es hat sich bewährt, dass die die Justiereinrichtung mit der Platte durch ein Gewinde verbunden ist, wobei eine Schraubensicherung, insbesondere ein Kunststoffteil, vorgesehen ist, welche eine Reibung im Gewinde erhöht, um ein Lösen der Justiereinrichtung zu verhindern. Somit ist ein unbeabsichtigtes Lösen der Justiereinrichtung von der Platte auf einfache Weise vermieden.

Um Fehler bei einem Einbau der Vorrichtung durch zu weites Einschrauben auf einfache Weise zu vermeiden, ist bevorzugt vorgesehen, dass die Justiereinrichtung nur bis zu einer vordefinierten Position in die Platte einschraubbar ist, wobei dies konstruktiv insbesondere mit einem Schraubenschlüssel, vorzugsweise einem Innensechskantschlüssel, umgesetzt ist, der ab einer vordefinierten Position der Justiereinrichtung keinen Kontakt mehr zu einem korrespondierenden Gegenstück, insbesondere einem Innensechskant, in der Justiereinrichtung herstellt.

Günstig ist es, wenn die Koppeleinrichtung zwischen einer medialen Endposition und einer lateralen Endposition zwischen Anschlägen bewegbar ist, wobei die mediale Endposition durch eine erste Anschlagfläche an der Koppeleinrichtung im Zusammenwirken mit der Justiereinrichtung und die laterale Endposition durch eine zweite Anschlagfläche im Zusammenwirken mit der Justiereinrichtung definiert wird. Die Justiereinrichtung definiert somit die Anschläge, welche eine Bewegbarkeit der Koppeleinrichtung auf einfache Weise begrenzt. Somit können sowohl eine Lage der Endpositionen als auch ein Spiel einfach eingestellt werden.

Es hat sich bewährt, dass die erste Anschlagfläche und die zweite Anschlagfläche unter einem Anschlagflächenwinkel zueinander, insbesondere unter unterschiedlichen Winkeln zu einer Justierrichtung, entlang welcher die Justiereinrichtung relativ zur Platte bewegbar ist, angeordnet sind, insbesondere unter einem Anschlagflächenwinkel zueinander von 10 Grad bis 160 Grad, vorzugsweise 60 Grad bis 120 Grad, sodass eine Bewegung der Justiereinrichtung entlang der Justierrichtung unterschiedlich starke Auswirkungen auf die laterale Endposition und die mediale Endposition hat. Somit kann durch eine Positionsänderung der Justiereinrichtung nicht nur eine Lage der Anschläge verändert werden, sondern auch ein Spiel, also eine Bewegbarkeit der Koppeleinrichtung zwischen den Anschlägen. Insbesondere wenn eine Anschlagfläche etwa parallel zur Justierrichtung ist, verändert eine Position der Justiereinrichtung entlang der Justierrichtung den durch die entsprechende Anschlagfläche gebildeten Anschlag bzw. die entsprechende Endposition nicht.

Wenn somit beispielsweise die mediale Endposition durch eine erste Anschlagfläche im Zusammenwirken mit der Justiereinrichtung definiert wird, welche erste Anschlagfläche etwa parallel zur Justierrichtung ist, ändert eine Positionsänderung der Justiereinrichtung entlang der Justierrichtung die mediale Endposition nicht.

Günstig ist es, wenn die zweite Anschlagfläche etwa normal zur Justierrichtung ist und die erste Anschlagfläche etwa parallel zur Justierrichtung ist, wobei insbesondere die Justierrichtung unter einem Winkel zu einer Längsachse der Koppeleinrichtung von 0,5 Grad bis 15 Grad, vorzugsweise 0,8 Grad bis 2 Grad, ausgerichtet ist. Somit bewirkt eine Bewegung der Justiereinrichtung entlang der Justierrichtung eine Verschiebung des zweiten Anschlages, jedoch nicht des ersten Anschlages, sodass ein Spiel auf einfache Weise veränderbar ist.

Besonders bevorzugt ist vorgesehen, dass die Justiereinrichtung als Justierschraube und die Koppeleinrichtung als Knochenschraube ausgebildet sind, wobei eine Längsachse der Justierschraube unter einem Winkel von 0,5 Grad bis 15 Grad, insbesondere 0,8 Grad bis 2 Grad, zu einer Längsachse der Knochenschraube ausgerichtet ist. Durch ein Zusammenwirken dieser beiden Schrauben ist somit ein Spiel auf besonders einfache Weise veränderbar. Gleichzeitig ist eine robuste und einfach handhabbare Ausführung gegeben. Üblicherweise sind die Justierschraube und die Knochenschraube aneinander angrenzend angeordnet und ragen vorzugsweise durch eine gemeinsame Öffnung in der Platte. Dadurch können Anschlagflächen zwischen Justierschraube und Knochenschraube, welche ein Spiel der Knochenschraube relativ zur Platte begrenzen, konstruktiv einfach und platzsparend umgesetzt werden.

Es hat sich bewährt, dass die Justiereinrichtung als Justierschraube ausgebildet ist, welche in einem Gewinde in der Platte angeordnet und mittels des Gewindes variabel positionierbar ist. Das Gewinde ist bevorzugt selbsthemmend. Die Justiereinrichtung ist somit in Lateralrichtung bzw. Medialrichtung nur durch eine Rotation, jedoch nicht durch einen Druck in Lateralrichtung bzw. Medialrichtung bewegbar. Dadurch kann das Spiel besonders genau eingestellt werden.

Bevorzugt ist somit die Justiereinrichtung entlang einer Justierrichtung, insbesondere entlang einer Gewindeachse, relativ zur Platte veränderbar positionierbar.

Besonders günstig ist es, wenn die Justierrichtung unter einem Winkel zu einer Längsachse der Koppeleinrichtung von 0,5 Grad bis 10 Grad, insbesondere 0,8 Grad bis 2 Grad, ausgerichtet ist, wobei vorzugsweise die Justierrichtung und eine Längsachse der Koppeleinrichtung in einer Ebene liegen. Dies ermöglicht eine besonders genaue Einstellbarkeit des Spiels. Es kann dann konstruktiv einfach insbesondere eine erste Anschlagfläche erreicht werden, an welcher die Koppeleinrichtung in einer medialen Endposition an der Justiereinrichtung anliegt, welche unter einem entsprechenden Winkel zu einer Längsrichtung der Koppeleinrichtung ausgerichtet ist und an welcher die Koppeleinrichtung an der Justiereinrichtung anliegt, sodass hier aufgrund des geringen Winkels eine besonders geringe Flächenpressung erreichtwird. Dadurch ist eine besonders hohe Stabilität gegeben.

Bevorzugt ist vorgesehen, dass die Koppeleinrichtung eine Schulter aufweist, welche insbesondere an einen abgeschrägten Bereich angrenzt, wobei die Schulter eine zweite Anschlagfläche bildet, die in einer lateralen Endposition der Koppeleinrichtung an der Justiereinrichtung anliegt, insbesondere etwa stirnseitig an der Justiereinrichtung. Somit ist ein Spiel über die beiden Anschlagflächen eindeutig definiert.

Besonders günstig ist es, wenn die beiden Anschlagflächen unter unterschiedlichen Winkeln zu einer Justierrichtung der Justierschraube angeordnet sind, entlang welcher Justierrichtung die Justierschraube relativ zur Platte bewegbar ist. Dadurch wirkt sich ein Bewegen der Justierschraube entlang der Justierrichtung unterschiedlich auf eine mediale Endposition und eine laterale Endposition der Koppeleinrichtung aus, sodass das Spiel auf einfache Weise eingestellt werden kann.

Die Justiereinrichtung ist bevorzugt als Justierschraube ausgebildet und weist somit in der Regel eine etwa zylindrische Hüllfläche auf. Mit einer entsprechenden abgeschrägten Fläche am Koppelelement ergibt sich somit ein Linienkontakt zwischen der Justiereinrichtung und der Koppeleinrichtung im Bereich der abgeschrägten Fläche, welche als erste Anschlagfläche dienen kann, die schräg, also nicht normal, zu einer Bewegungsrichtung der Koppeleinrichtung entlang der Längsachse der Koppeleinrichtung ausgerichtet ist. Dadurch ergibt sich eine gute Kraftübertragung an der ersten Anschlagfläche. Wenn die zweite Anschlagfläche an einer Schulter bzw. einem Übergang vom abgeschrägten Bereich zu einem daran anschließenden beispielsweise zylindrischen Bereich positioniert ist, kann die zweite Anschlagfläche etwa normal zur Längsachse der Koppeleinrichtung ausgerichtet sein, sodass eine Bewegung der Justiereinrichtung entlang der Justierrichtung unterschiedlich starke Auswirkungen auf eine Lage von medialer Endposition, welche in der Regel durch die erste Anschlagfläche im Zusammenwirken mit der Justiereinrichtung bewirkt wird, und laterale Endposition, welche in der Regel durch die zweite Anschlagfläche im Zusammenwirken mit der Justiereinrichtung bewirkt wird, hat. Dadurch kann das Spiel einfach durch ein Verändern einer Position der Justiereinrichtung entlang der Justierrichtung verändert werden, obwohl beide Anschlagflächen mit der Justiereinrichtung zusammenwirken.

Der zylindrische Bereich der Koppeleinrichtung schließt üblicherweise medial an den konischen Bereich an, sodass sich zwischen konischem Bereich und zylindrischem Bereich eine Schulter ergibt, die als zweite Anschlagfläche nutzbar ist, um eine Bewegbarkeit der Koppeleinrichtung in Lateralrichtung relativ zur Platte zu begrenzen.

Günstig ist es, wenn die Koppeleinrichtung einen abgeschrägten Bereich aufweist und die Justiereinrichtung eine zumindest bereichsweise etwa zylindrische Außenkontur aufweist, wobei der abgeschrägte Bereich an der Koppeleinrichtung eine erste Anschlagfläche bildet, welche im Zusammenwirken mit der etwa zylindrischen Außenkontur der Justiereinrichtung eine Bewegbarkeit der Koppeleinrichtung relativ zur Platte in Medialrichtung begrenzt. Eine zylindrische Außenkontur der Justiereinrichtung ergibt sich beispielsweise, wenn die Justiereinrichtung durch eine Justierschraube gebildet ist. Dies gewährleistet eine besonders gute Kraftübertragung zwischen Koppeleinrichtung und Justiereinrichtung und ermöglicht einen einfach und robust einstellbaren Anschlag, um ein Spiel zu definieren.

Besonders bevorzugt ist vorgesehen, dass der abgeschrägte Bereich unter einem Abschrägungswinkel zu einer Längsachse der Koppeleinrichtung ausgerichtet ist, welcher mit einem Winkel korrespondiert, unter welchem die Justiereinrichtung zur Längsachse der Koppeleinrichtung ausgerichtet ist und die Justiereinrichtung derart angeordnet ist, dass die Justiereinrichtung den abgeschrägten Bereich berührt. Die Justiereinrichtung kann dann entlang der Justierrichtung in die Platte eingeschraubt werden, ohne mit der Koppeleinrichtung zu kollidieren, sodass eine Lage einer lateralen Endposition leicht veränderbar ist. Eine mediale Endposition ergibt sich dann in der Regel durch Abmessungen von Justiereinrichtung und Koppeleinrichtung und kann unabhängig von einer Position der Justiereinrichtung entlang der Justierrichtung sein.

Günstig ist es, wenn eine Zugschraube vorgesehen ist, welche etwa parallel zu einer Längsachse der Koppeleinrichtung angeordnet ist, insbesondere proximal zur Koppeleinrichtung, mit welcher Zugschraube die Platte an einer Zugschraubenöffnung durch den Oberschenkelschaft und einen Schenkelhals hindurch mit dem Oberschenkelkopf verbindbar ist, um eine Rotation des Oberschenkelkopfes relativ zur Koppeleinrichtung zu verhindern.

Üblicherweise ist vorgesehen, dass eine Längsachse der Koppeleinrichtung unter einem Schenkelhalswinkel von 10 Grad bis 60 Grad, insbesondere 35 Grad bis 45 Grad, zu einer Längsachse der ersten Schraube ausgerichtet ist. Die entsprechenden Winkel ergeben sich durch Anordnung und Ausrichtung der Koppeleinrichtungsöffnung und der ersten Öffnungen in der Platte und sind dann günstig, um Oberschenkelhalsfrakturen zu versorgen, zumal ein Winkel zwischen einem Schenkelhals und einem Oberschenkelschaft üblicherweise im Bereich von etwa 130 Grad liegt.

Bevorzugt ist vorgesehen, dass die Koppeleinrichtung hohl ausgebildet ist, sodass Knochenzement durch die Koppeleinrichtung im Bereich des Oberschenkelkopfes einbringbar ist, wenn die Vorrichtung an einem Oberschenkelknochen angeordnet ist.

Bevorzugt ist auch die Zugschraube hohl ausgebildet, um gegebenenfalls auch durch diese hindurch Knochenzement in den Bereich des Oberschenkelkopfes einbringen zu können.

Weitere Merkmale, Vorteile und Wirkungen der Erfindung ergeben sich anhand des nachfolgend dargestellten Ausführungsbeispiels. In den Zeichnungen, auf welche dabei Bezug genommen wird, zeigen:
Fig. 1 und 2 eine erfindungsgemäße Vorrichtung in unterschiedlichen Ansichten;
Fig. 2a ein Detail der Fig. 1;
Fig. 3 und 4 ein Detail der Vorrichtung in unterschiedlichen Betriebszuständen;
Fig. 5 und 6 Darstellungen, welche das Funktionsprinzip der Vorrichtung veranschaulichen;
Fig. 7 und 8 Details einer weiteren erfindungsgemäßen Vorrichtung.

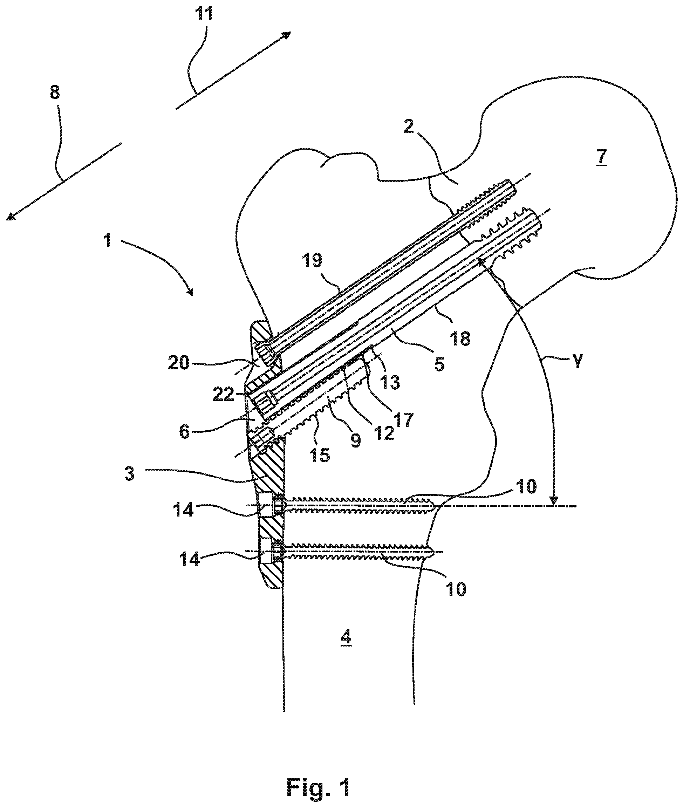

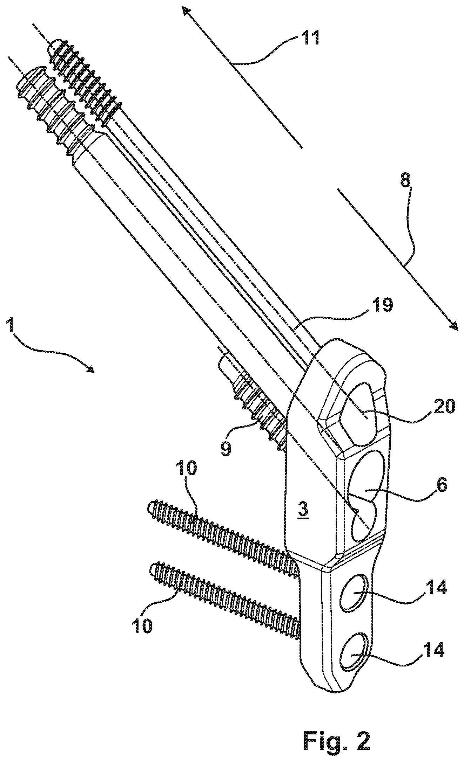

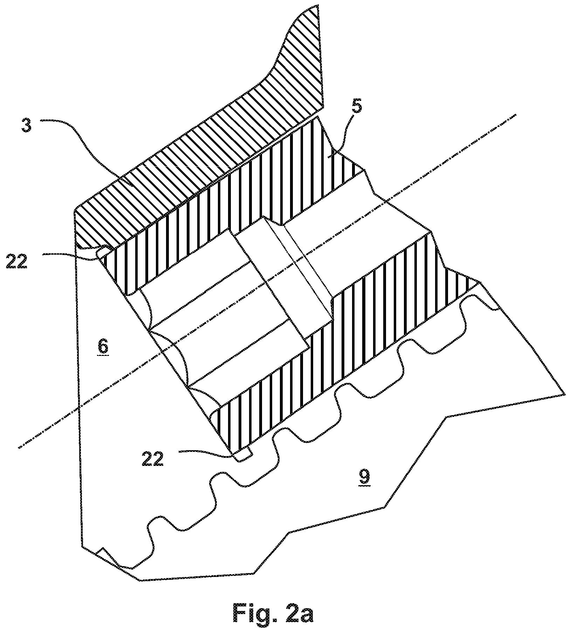

Fig. 1 zeigt eine erfindungsgemäße Vorrichtung 1 in Schnittdarstellung an einem Oberschenkelknochen mit einem Oberschenkelschaft 4, einem Schenkelhals 2 und einem Oberschenkelkopf 7. In Fig. 2 wird diese Vorrichtung 1 ohne Oberschenkelknochen in einer 3D-Ansicht dargestellt.

Ersichtlich ist jeweils, dass eine Platte 3 vorgesehen ist, welche außenseitig an einem Oberschenkelknochen anbringbar ist, und zwar gegenüber einem Schenkelhals 2, und durch Intraossärschrauben 10, welche durch Intraossärschraubenöffnungen 14 in der Platte 3 ragen, in einem unteren Bereich mit dem Oberschenkelschaft 4 verbunden werden kann.

Oberhalb der Intraossärschrauben 10 bzw. proximal dazu sind eine Koppeleinrichtung, welche hier durch eine Knochenschraube 5 gebildet ist, und eine Zugschraube 19 vorgesehen, welche als Schaftschrauben ausgebildet sind und nur in einem endseitigen Bereich ein Gewinde 15 aufweisen. Dadurch ist es möglich, den Oberschenkelkopf 7, in welchen diese Schrauben geschraubt werden, an den Schenkelhals 2 bzw. den Oberschenkelschaft 4 zu ziehen und somit eine Stabilisierung der Fraktur zu erreichen. Hierzu sind in der Platte 3 eine Koppeleinrichtungsöffnung 6, durch welche die Knochenschraube 5 ragt, und eine Zugschraubenöffnung 20 vorgesehen, an welcher die Zugschraube 19 die Platte 3 durchdringt. Die Knochenschraube weist an einem lateralen Ende einen Bund 22 auf, welcher an der Koppeleinrichtungsöffnung 6 anliegt und somit einen Anschlag bildet, bis zu welchem die Knochenschraube medial in die Platte eingeführt werden kann.

Die Knochenschraube 5 ist mit einem Spiel 21 in Medialrichtung 11 bzw. Lateralrichtung 8 mit der Platte 3 verbunden. Das Spiel 21 ergibt sich durch eine Bewegbarkeit der Koppeleinrichtung relativ zur Platte 3 zwischen einem ersten Anschlag und einem zweiten Anschlag, welche sich jeweils an einer hier durch eine Justierschraube 9 gebildeten Justiereinrichtung befinden. Die Justierschraube 9 weist ein Gewinde 15 auf, welches mit einem Gewinde 15 in der Platte 3 korrespondiert, sodass die Justierschraube 9 entlang einer Justierrichtung 16, etwa in Medialrichtung 11, die Platte 3 eingeschraubt bzw. entgegengesetzt, etwa in Lateralrichtung 8, aus der Platte 3 geschraubt werden kann, um ein Spiel 21 der Knochenschraube 5 relativ zur Platte 3, und somit eine Bewegbarkeit des Oberschenkelkopfes 7 relativ zum Schenkelhals 2, zu beeinflussen.

Der erste Anschlag wird gebildet durch eine erste Anschlagfläche 12 an der Knochenschraube 5 in einem abgeschrägten Bereich 17, welche erste Anschlagfläche 12 in einer medialen Endposition der Knochenschraube 5 umfangsseitig an der Justierschraube 9 anliegt. So ist die Justierrichtung 16 nicht parallel zu einer Längsachse der Knochenschraube 5, sondern unter einem Winkel α von etwa 1,2 Grad dazu angeordnet. Der abgeschrägte Bereich 17 an der Knochenschraube 5 ist etwa parallel zur Justierrichtung 16, eine Normale auf den abgeschrägten Bereich 17 ist somit etwa normal auf die Justierrichtung 16, sodass die mediale Endposition unabhängig von einer Einschraubtiefe der Justierschraube 9 ist. Ferner liegt die Knochenschraube 5 in der medialen Endposition über den am lateralen Ende vorgesehenen Bund 22 an der Platte 3 an, sodass auch dieser Bund 22 einen Anschlag bildet bzw. die mediale Endposition definiert.

Der zweite Anschlag wird durch eine zweite Anschlagfläche 13 in einem Bereich einer Schulter der Knochenschraube 5 gebildet, welche sich in einem Bereich zwischen dem abgeschrägten Bereich 17 und einem zylindrischen Bereich 18 der Knochenschraube 5 ergibt. Diese Schulter liegt in einer lateralen Endposition der Knochenschraube 5 an der Justierschraube 9 stirnseitig an. Die laterale Endposition ändert sich somit mit der Einschraubtiefe der Justierschraube 9 entlang der Justierrichtung 16. Es ergibt sich somit durch eine Veränderung der Position der Justierschraube 9 relativ zur Platte 3 eine Änderung der lateralen Endposition der Knochenschraube 5 und somit eine Änderung des Spiels 21.

Proximal zur Knochenschraube 5 ist eine weitere Zugschraube 19 vorgesehen, welche ebenfalls von der Platte 3 bis in den Schenkelkopf geschraubt wird. Diese zusätzliche Zugschraube 19 stabilisiert einerseits den Oberschenkelkopf 7 am Schenkelhals 2 und verhindert andererseits eine Rotation des Oberschenkelkopfes 7 um die Knochenschraube 5 bzw. die Koppeleinrichtung.

Wie ersichtlich sind die Knochenschraube 5 und die Zugschraube 19 etwa parallel und unter einem Schenkelhalswinkel γ von etwa 20 Grad bis 40 Grad zu den Intraossärschrauben 10 ausgerichtet.

Fig. 2a zeigt ein vergrößertes Detail der Fig. 1. Hier ist besonders der umlaufende Bund 22 gut erkennbar, welcher in der medialen Endposition der Knochenschraube 5 an der Platte 3 anliegt und somit einen Anschlag bildet, bis zu welchem die Knochenschraube 5 in die Platte 3 eingeführt werden kann. Die Koppeleinrichtungsöffnung 6 ist wie ersichtlich kleiner als der Bund 22, jedoch groß genug, damit die Knochenschraube 5 bis zum Bund 22 durch die Koppeleinrichtungsöffnung 6 in die Platte 3 eingeführt werden kann.

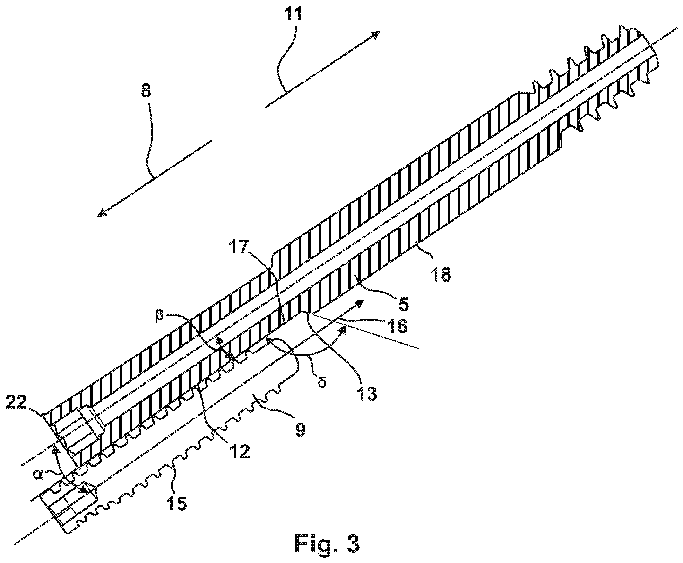

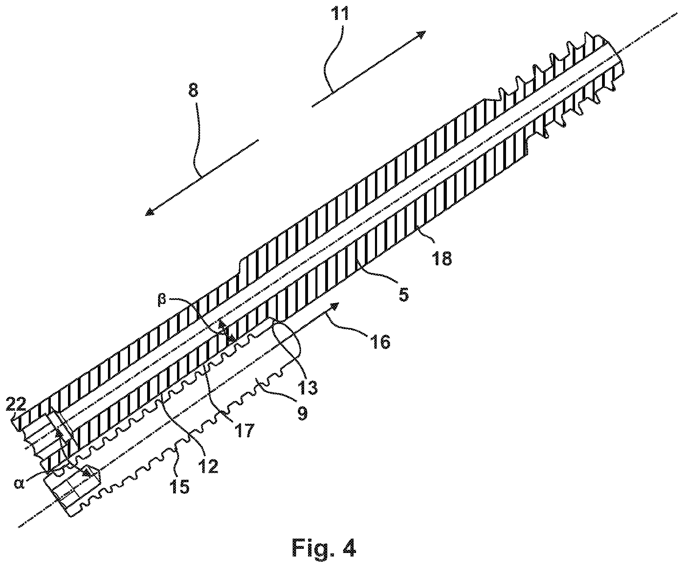

Fig. 3 und 4 zeigen die Knochenschraube 5 und die Justierschraube 9 der in Fig. 1 und 2 dargestellten Vorrichtung 1 im Detail. Fig. 3 zeigt die Knochenschraube 5 und die Justierschraube 9 dabei in einer medialen Endposition der Knochenschraube 5, wenn also der Oberschenkelkopf 7 maximal vom Schenkelhals 2 beabstandet ist. Wie ersichtlich ist dann keine weitere Bewegung der Knochenschraube 5 in medialer Richtung möglich, da die Knochenschraube 5 am abgeschrägten Bereich 17, welcher die erste Anschlagfläche 12 bildet, an einem Umfangsbereich der Justierschraube 9 anliegt. Eine Bewegung der Knochenschraube 5 und des Oberschenkelkopfes 7, in welchen die Knochenschraube 5 endseitig üblicherweise eingeschraubt ist, entgegengesetzt, also in Lateralrichtung 8, wäre aus dieser Position heraus allerdings möglich.

Wie hier gut ersichtlich sind die erste Anschlagfläche 12 und die zweite Anschlagfläche 13 unter einem Anschlagflächenwinkel δ von etwa 110 Grad relativ zueinander angeordnet. Die erste Anschlagfläche 12 ist dabei etwa parallel zur Justierrichtung 16 orientiert, sodass die zweite Anschlagfläche 13 unter einem Winkel α von etwa 110 Grad zur Justierrichtung 16 orientiert ist. Dadurch bewirkt eine Veränderung einer Position der Justierschraube 9 entlang der Justierrichtung 16 nur eine Änderung der lateralen Endposition, jedoch keine Änderung der medialen Endposition, welche zudem durch den Bund 22 bestimmt ist.

Fig. 4 zeigt die Knochenschraube 5 und die Justierschraube 9 in einer lateralen Endposition der Knochenschraube 5, wenn also der Oberschenkelkopf 7 nicht näher an den Schenkelhals 2 bewegt werden kann, somit also keine weitere Bewegung der Knochenschraube 5 in Lateralrichtung möglich ist. Wie ersichtlich liegt die Knochenschraube 5 in dieser lateralen Endposition an einer durch eine Schulter an der Knochenschraube 5 gebildeten zweiten Anschlagfläche 13 etwa stirnseitig an der Justierschraube 9 an.

Ein Abschrägungswinkel β, unter welchem der abgeschrägte Bereich 17 zu einer Längsachse der Knochenschraube 5 ausgerichtet ist, entspricht etwa einem Winkel α, unter welchem die Justierrichtung 16 zur Längsachse der Knochenschraube ausgerichtet ist, sodass der abgeschrägte Bereich 17 etwa parallel zur Justierrichtung 16 ist. Dadurch hat eine Änderung der Einschraubtiefe der Justierschraube 9 keine Auswirkung auf eine Lage der medialen Endposition. Diese ist vielmehr ausschließlich durch Abmessungen von Knochenschraube 5 und Justierschraube 9 und den Winkel α bedingt. Allerdings ändert sich mit der Einschraubtiefe der Justierschraube 9 eine Lage der lateralen Endposition, da diese von einer Position einer Stirnseite der Justierschraube 9 abhängig ist. Durch eine Änderung der Einschraubtiefe der Justierschraube 9 kann daher über die laterale Endposition ein Spiel 21 einfach eingestellt werden, um welches die Knochenschraube 5 relativ zur Platte 3 bewegbar ist. Diese Einstellbarkeit ist für eine rasche Heilung einer entsprechenden Fraktur besonders wichtig.

Wie in Fig. 1, 3 und 4 ersichtlich ist die Knochenschraube 5 hohl ausgebildet, sodass durch diese Knochenzement bis in einen Bereich eines Oberschenkelkopfes 7 eingebracht werden kann, um die Knochenschraube 5 beispielsweise dort zu fixieren.

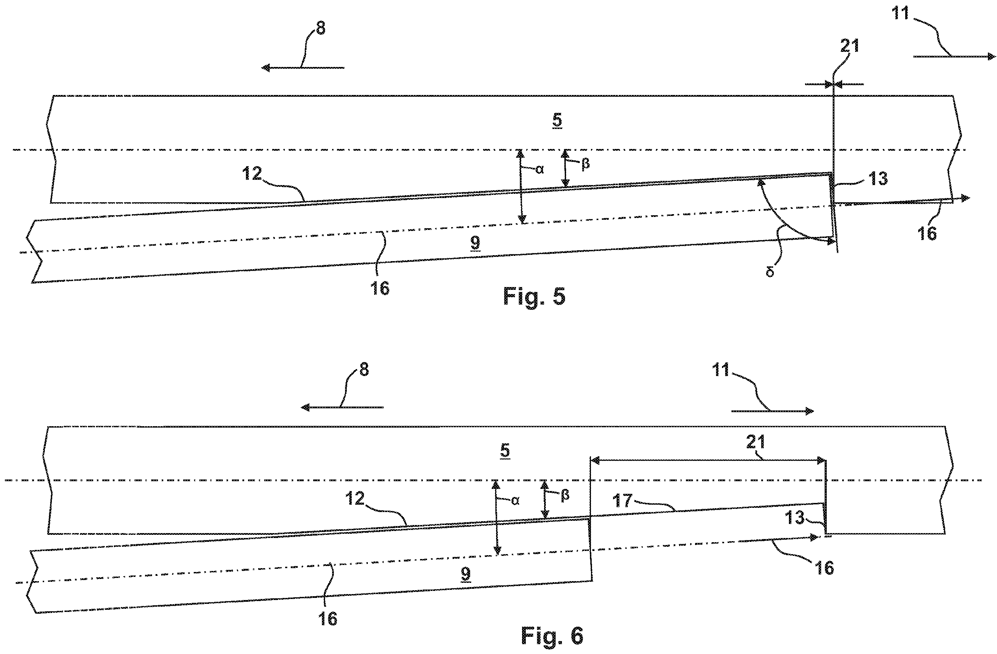

Fig. 5 und 6 zeigen in dem Zusammenhang einen Einfluss einer Einschraubtiefe der Justierschraube 9 auf das Spiel 21, wobei Abmessungen und Winkel α zur besseren Veranschaulichung nicht maßstäblich dargestellt sind. Wie ersichtlich ändert sich aufgrund des zur Justierrichtung 16 etwa parallelen abgeschrägten Bereiches 17 der Knochenschraube 5 die erste Anschlagfläche 12 bzw. eine mediale Endposition mit einer Einschraubtiefe der Justierschraube 9 entlang der Justierrichtung 16 nicht, jedoch hat die Einschraubtiefe unmittelbare Auswirkungen auf die zweite Anschlagfläche 13, welche die laterale Endposition definiert. So zeigt Fig. 5 eine Situation, bei welcher die Justierschraube 9 weit in die nicht dargestellte Platte 3 eingeschraubt ist, sodass kein Spiel 21 verbleibt. Die Knochenschraube 5 liegt in dieser Situation sowohl an der ersten Anschlagfläche 12 als auch an der zweiten Anschlagfläche 13 an. In der in Fig. 6 dargestellten Situation ist die Justierschraube 9 lateral weiter außen angeordnet bzw. nicht so weit wie in Fig. 5 dargestellt in die Platte 3 eingeschraubt.

Die als Justierschraube 9 ausgebildete Justiereinrichtung ist unter einem Winkel α von etwa 1,2 Grad zur Längsachse der Knochenschraube 5 ausgerichtet. Entsprechend ist auch ein Kegelwinkel des konischen Bereiches ausgebildet, sodass sich ein Linienkontakt zwischen der Justierschraube 9 und dem konischen Bereich der Knochenschraube 5 ergibt, wodurch eine besonders gute Stabilisierung und Kraftübertragung gewährleistet sind. Auch hier ist ersichtlich, dass die erste Anschlagfläche 12 unter einem Anschlagflächenwinkel δ zur zweiten Anschlagfläche 13 ausgerichtet ist, welcher hier etwa 90 Grad beträgt, sodass eine Positionsänderung der Justierschraube 9 unterschiedlich große Auswirkungen auf die mediale Endposition und die laterale Endposition hat.

Wenngleich in den Ausführungsbeispielen an einer Verbindung zwischen der ersten Anschlagfläche 12 und der zweiten Anschlagfläche 13 eine Ecke bzw. Kante dargestellt ist, versteht es sich, dass die erste Anschlagfläche 12 und die zweite Anschlagfläche 13 auch beispielsweise über eine Verrundung verbunden sein und dennoch einen entsprechenden Anschlagflächenwinkel δ relativ zueinander aufweisen könnten. Die Knochenschraube 5 kann daher beispielsweise sowohl unter Verwendung eines Kugelfräsers als auch unter Verwendung eines Schaftfräsers oder dergleichen auf einfache Weise hergestellt werden.

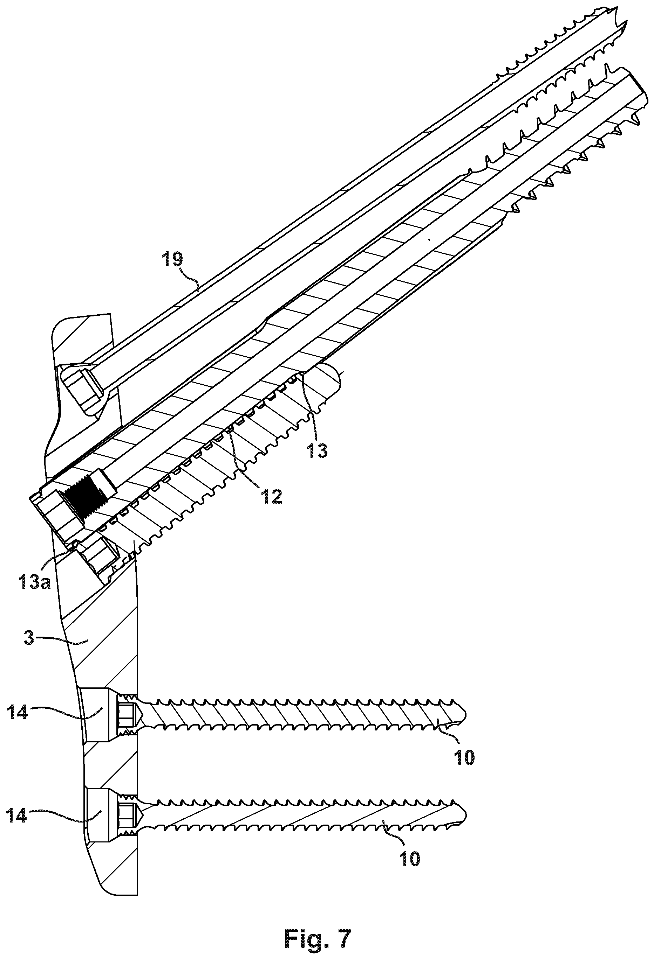

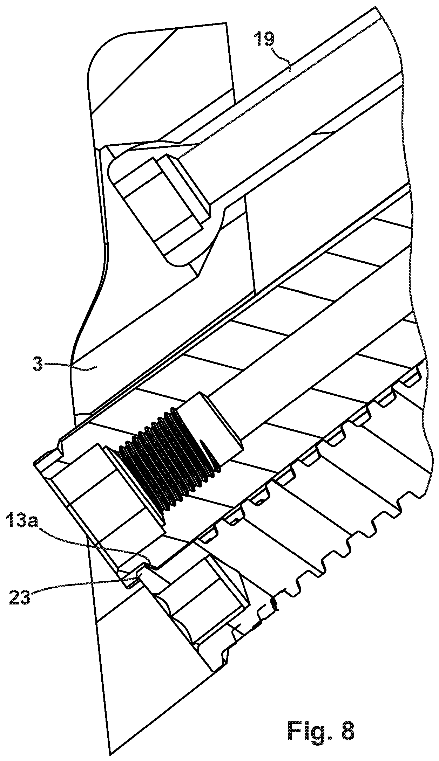

Fig. 7 und 8 zeigen Details eines weiteren Ausführungsbeispiels einer erfindungsgemäßen Vorrichtung 1. Diese Vorrichtung 1 weist eine durch zwei Kontaktflächen, die parallel wirken gebildete zweite Anschlagfläche 13 auf, nämlich einen primäre zweite Anschlagfläche 13 an der Koppeleinrichtung, welche in der lateralen Endposition an einem medialen Gewindeende der Justiereinrichtung anliegt, und einer sekundären zweiten Anschlagfläche 13a an einem lateralen Ende der Justiereinrichtung, welche sekundäre zweite Anschlagfläche 13a wie in Fig. 8 im Detail ersichtlich durch einen Justierschraubenkragen 23, also einen Bereich der Justierschraube 9 mit größerem Durchmesser als die übrige Justierschraube 9, in Verbindung mit einer endseitige Verjüngung bzw. umlaufenden Vertiefung an der Koppeleinrichtung, also hier der Knochenschraube 5, gebildet wird. Durch die beiden zweiten Anschlagflächen 13, 13a, welche parallel wirken, ist ein unbeabsichtigtes Gleiten der Justierschraube 9 entlang der Koppeleinrichtung auf einfache Weise verhindert und somit eine Bewegbarkeit zuverlässig begrenzt.

Eine erfindungsgemäße Vorrichtung 1 ermöglicht die Versorgung einer Fraktur eines Oberschenkelknochens auf besonders einfache Weise und gewährleistet aufgrund des einfach einstellbaren Spiels 21 eine besonders gute Heilung.

## Patentansprüche

1. Vorrichtung (1) zum Versorgen einer Fraktur, insbesondere einer Fraktur eines proximalen Femurs wie beispielsweise einer Schenkelhalsfraktur, aufweisend eine Platte (3) zur Anbringung an einem Oberschenkelknochen, eine oder mehrere Intraossärschrauben (10), mit welchen die Platte (3) durch Intraossärschraubenöffnungen (14) hindurch an einem Oberschenkelschaft (4) fixierbar ist, eine proximal zu der zumindest einen Intraossärschraube (10) angeordnete Koppeleinrichtung, insbesondere eine Knochenschraube (5), mit welcher die Platte (3) an einer Koppeleinrichtungsöffnung (6) durch den Oberschenkelschaft (4) und einen Schenkelhals (2) hindurch mit einem Oberschenkelkopf (7) verbindbar ist, wobei die Koppeleinrichtung mit einem Spiel (21) mit der Platte (3) verbunden ist, sodass die Koppeleinrichtung in Lateralrichtung (8) relativ zur Platte (3) bewegbar ist, wobei eine mit der Platte (3) verbundene und an unterschiedlichen Positionen relativ zur Platte (3) positionierbare Justiereinrichtung vorgesehen ist, mit welcher das Spiel (21) einstellbar ist, **dadurch gekennzeichnet, dass** die Koppeleinrichtung zwischen einer medialen Endposition und einer lateralen Endposition zwischen Anschlägen bewegbar ist, wobei die mediale Endposition durch eine erste Anschlagfläche (12) an der Koppeleinrichtung im Zusammenwirken mit der Justiereinrichtung und die laterale Endposition durch eine zweite Anschlagfläche (13) im Zusammenwirken mit der Justiereinrichtung definiert wird.

2. Vorrichtung (1) nach Anspruch 1, **dadurch gekennzeichnet, dass** die Justiereinrichtung in Lateralrichtung (8) und Medialrichtung (11) im Wesentlichen starr mit der Platte (3) verbunden ist.

3. Vorrichtung (1) nach Anspruch 1 oder 2, **dadurch gekennzeichnet, dass** die Koppeleinrichtung eine erste Anschlagfläche (12) aufweist, welche mit einer korrespondierenden Fläche an der Justiereinrichtung und/oder der Platte (3) zusammenwirkt, sodass durch die erste Anschlagfläche (12) eine Bewegbarkeit der Koppeleinrichtung relativ zur Platte (3) in Medialrichtung (11) begrenzt ist.

4. Vorrichtung (1) nach einem der Ansprüche 1 bis 3, **dadurch gekennzeichnet, dass** die Koppeleinrichtung lateral einen Bund (22) aufweist, welcher im Zusammenwirken mit der Platte (3) einen Anschlag bildet, bis zu welchem die Koppeleinrichtung medial in die Platte (3) einführt werden kann.

5. Vorrichtung (1) nach einem der Ansprüche 1 bis 4, **dadurch gekennzeichnet, dass** die Koppeleinrichtung eine zweite Anschlagfläche (13) aufweist, welche in einer lateralen Endposition der Koppeleinrichtung an der Justiereinrichtung anliegt.

6. Vorrichtung (1) nach Anspruch 5, **dadurch gekennzeichnet, dass** die zweite Anschlagfläche (13) zumindest teilweise durch eine laterale zweite Anschlagfläche (13a) gebildet wird, welche in der lateralen Endposition zumindest teilweise an einer Schulter oder einem Kragen der Justiereinrichtung, insbesondere an einem Justierschraubenkragen (23), in einem Bereich eines lateralen Endes der Justiereinrichtung anliegt.

7. Vorrichtung (1) nach Anspruch 5 oder 6, **dadurch gekennzeichnet, dass** die zweite Anschlagfläche (13) zumindest teilweise durch eine mediale zweite Anschlagfläche an der Koppeleinrichtung gebildet wird, welche in der lateralen Endposition zumindest teilweise an einem Gewinde der Justiereinrichtung anliegt.

8. Vorrichtung (1) nach einem der Ansprüche 1 bis 7, **dadurch gekennzeichnet, dass** die Justiereinrichtung mit der Platte (3) durch ein Gewinde verbunden ist, wobei eine Schraubensicherung, insbesondere ein Kunststoffteil, vorgesehen ist, welche eine Reibung im Gewinde erhöht, um ein Lösen der Justiereinrichtung zu verhindern.

9. Vorrichtung (1) nach einem der Ansprüche 1 bis 8, **dadurch gekennzeichnet, dass** die Justiereinrichtung nur bis zu einer vordefinierten Position in die Platte (3) einschraubbar ist, wobei dies konstruktiv insbesondere mit einem Schraubenschlüssel, vorzugsweise einem Innensechskantschlüssel, umgesetzt ist, der ab einer vordefinierten Position der Justiereinrichtung keinen Kontakt mehr zu einem korrespondierenden Gegenstück, insbesondere einem Innensechskant, in der Justiereinrichtung herstellt.

10. Vorrichtung (1) nach einem der Ansprüche 1 bis 9, **dadurch gekennzeichnet, dass** die erste Anschlagfläche (12) und die zweite Anschlagfläche (13) unter einem Anschlagflächenwinkel (δ) zueinander, insbesondere unter unterschiedlichen Winkeln zu einer Justierrichtung (16), entlang welcher die Justiereinrichtung relativ zur Platte (3) bewegbar ist, angeordnet sind, insbesondere unter einem Anschlagflächenwinkel (δ) zueinander von 10 Grad bis 160 Grad, vorzugsweise 60 Grad bis 120 Grad, sodass eine Bewegung der Justiereinrichtung entlang der Justierrichtung (16) unterschiedlich starke Auswirkungen auf die laterale Endposition und die mediale Endposition hat.

11. Vorrichtung (1) nach Anspruch 10, **dadurch gekennzeichnet, dass** die zweite Anschlagfläche (13) etwa normal zur Justierrichtung (16) ist und die erste Anschlagfläche (12) etwa parallel zur Justierrichtung (16) ist, wobei insbesondere die Justierrichtung (16) unter einem Winkel (α) zu einer Längsachse der Koppeleinrichtung von 0,5 Grad bis 15 Grad, vorzugsweise 0,8 Grad bis 2 Grad, ausgerichtet ist.

12. Vorrichtung (1) nach einem der Ansprüche 1 bis 11, **dadurch gekennzeichnet, dass** die Justiereinrichtung als Justierschraube (9) und die Koppeleinrichtung als Knochenschraube (5) ausgebildet sind, wobei eine Längsachse der Justierschraube (9) unter einem Winkel (α) von 0,5 Grad bis 15 Grad, insbesondere 0,8 Grad bis 2 Grad, zu einer Längsachse der Knochenschraube (5) ausgerichtet ist.

13. Vorrichtung (1) nach einem der Ansprüche 1 bis 12, **dadurch gekennzeichnet, dass** die Justiereinrichtung als Justierschraube (9) ausgebildet ist, welche in einem Gewinde (15) in der Platte (3) angeordnet und mittels des Gewindes (15) variabel positionierbar ist.

14. Vorrichtung (1) nach einem der Ansprüche 1 bis 13, **dadurch gekennzeichnet, dass** die Justiereinrichtung entlang einer Justierrichtung (16), insbesondere einer Gewindeachse, relativ zur Platte (3) veränderbar positionierbar ist.

15. Vorrichtung (1) nach Anspruch 14, **dadurch gekennzeichnet, dass** die Justierrichtung (16) unter einem Winkel (α) zu einer Längsachse der Koppeleinrichtung von 0,5 Grad bis 10 Grad, insbesondere 0,8 Grad bis 2 Grad, ausgerichtet ist, wobei vorzugsweise die Justierrichtung (16) und eine Längsachse der Koppeleinrichtung in einer Ebene liegen.

## Claims

1. A device (1) for treating a fracture, in particular a fracture of a proximal femur such as, for example, a femoral neck fracture, having a plate (3) for attaching to a thigh bone, one or more intraosseous screws (10) by means of which the plate (3), through intraosseous screw openings (14), can be fixed to a thigh shaft (4), a coupling device, in particular a bone screw (5), which is arranged proximally to the at least one intraosseous screw (10), by means of which the plate (3) can be connected at a coupling device opening (6) through the thigh shaft (4) and a thigh neck (2) to a thigh head (7), wherein the coupling device is connected to the plate (3) with play (21) such that the coupling device can be moved in the lateral direction (8) relative to the plate (3), wherein an adjusting device is provided which is connected to the plate (3) and can be positioned at different positions relative to the plate (3), by means of which the play (21) can be adjusted, **characterized in that** the coupling device is movable between stops between a medial end position and a lateral end position, wherein the medial end position is defined by a first stop surface (12) on the coupling device in cooperation with the adjusting device, and the lateral end position is defined by a second stop surface (13) in cooperation with the adjusting device.

2. The device (1) according to Claim 1, **characterized in that** the adjusting device is connected to the plate (3) substantially rigidly in the lateral direction (8) and the medial direction (11).

3. The device (1) according to Claim 1 or 2, **characterized in that** the coupling device has a first stop surface (12), which cooperates with a corresponding surface on the adjusting device and/or the plate (3), so that a mobility of the coupling device relative to the plate (3) in medial direction (11) is limited by the first stop surface (12).

4. The device (1) according to one of Claims 1 to 3, **characterized in that** the coupling device has a collar (22) laterally which in cooperation with the plate (3) forms a stop, up to which the coupling device can be introduced medially into the plate (3).

5. The device (1) according to one of Claims 1 to 4, **characterized in that** the coupling device has a second stop surface (13), which lies against the adjusting device when the coupling device is in a lateral end position.

6. The device (1) according to Claim 5, **characterized in that** the second stop surface (13) is formed at least partly by a lateral second stop surface (13a) which in the lateral end position at least partly lies against a shoulder or flange of the adjusting device, in particular against an adjusting screw flange (23), in a region of a lateral end of the adjusting device.

7. The device (1) according to Claim 5 or 6, **characterized in that** the second stop surface (13) is formed at least partly by a medial second stop surface on the coupling device, which at least partly lies against a thread of the adjusting device in the lateral end position.

8. The device (1) according to one of Claims 1 to 7, **characterized in that** the adjusting device is connected to the plate (3) by a thread, wherein a screw lock, in particular a plastic part is provided, which increases a friction in the thread in order to prevent the adjusting device from becoming detached.

9. The device (1) according to one of Claims 1 to 8, **characterized in that** the adjusting device can only be screwed into the plate (3) as far as a predefined position, wherein this is implemented structurally in particular with a wrench, preferably an Allen wrench, which no longer produces a contact with a corresponding counterpart, in particular an internal hexagon in the adjusting device, beyond a predefined position of the adjusting device.

10. The device (1) according to one of Claims 1 to 9, **characterized in that** the first stop surface (12) and the second stop surface (13) are arranged at a stop surface angle (δ) relative to one another, in particular at different angles to an adjustment direction (16), along which the adjusting device is movable relative to the plate (3), in particular at a stop surface angle (δ) relative to one another from 10 degrees to 160 degrees, preferably 60 degrees to 120 degrees, so that a movement of the adjusting device along the adjustment direction (16) has effects of different extent on the lateral end position and the medial end position.

11. The device (1) according to Claim 10, **characterized in that** the second stop surface (13) is approximately normal to the adjustment direction (16), and the first stop surface (12) is approximately parallel to the adjustment direction (16), wherein in particular the adjustment direction (16) is aligned at an angle (α) to a longitudinal axis of the coupling device from 0.5 degrees to 15 degrees, preferably 0.8 degrees to 2 degrees.

12. The device (1) according to one of Claims 1 to 11, **characterized in that** the adjusting device is embodied as adjusting screw (9) and the coupling device is embodied as bone screw (5), wherein a longitudinal axis of the adjusting screw (9) is aligned at an angle (α) from 0.5 degrees to 15 degrees, in particular 0.8 degrees to 2 degrees, to a longitudinal axis of the bone screw (5).

13. The device (1) according to one of Claims 1 to 12, **characterized in that** the adjusting device is embodied as adjusting screw (9), which is arranged in a thread (15) in the plate (3) and is able to be positioned variably by means of the thread (15).

14. The device (1) according to one of Claims 1 to 13, **characterized in that** the adjusting device is able to be positioned in a changeable manner along an adjustment direction (16), in particular along a thread axis, relative to the plate (3).

15. The device (1) according to Claim 14, **characterized in that** the adjustment direction (16) is aligned at an angle (α) from 0.5 degrees to 10 degrees, in particular 0.8 degrees to 2 degrees relative to a longitudinal axis of the coupling device, wherein preferably the adjustment direction (16) and a longitudinal axis of the coupling device lie in one plane.

## Revendications

1. Dispositif (1) permettant de soigner une fracture, en particulier une fracture d'un fémur proximal, telle qu'une fracture du col du fémur, présentant une plaque (3) pour fixation à un fémur, une ou plusieurs vis intra-osseuses (10) permettant de fixer la plaque (3) à travers des ouvertures de vis intra-osseuses (14) à une tige de fémur (4), un dispositif de couplage proximal à l'au moins une vis intra-osseuse (10), en particulier une vis à os (5), avec laquelle la plaque (3) peut être connectée au niveau d'une ouverture de dispositif de couplage (6) par l'intermédiaire de la tige de fémur (4) et d'un col de fémur (2), à la tête de fémur (7), le dispositif de couplage étant relié à la plaque (3) avec un jeu (21), de sorte que le dispositif de couplage soit mobile dans le sens latéral (8) par rapport à la plaque (3), étant prévu un dispositif de réglage relié à la plaque (3) et positionnable à différentes positions par rapport à la plaque (3) avec lequel le jeu (21) peut être réglé, **caractérisé en ce que** le dispositif de couplage peut être déplacé entre une position terminale médiale et une position terminale latérale entre des butées, la position terminale médiale étant définie par une première surface de butée (12) sur le dispositif de couplage en interaction avec le dispositif de réglage et la position terminale latérale par une seconde surface de butée (13) en interaction avec le dispositif de réglage.

2. Dispositif (1) selon la revendication 1, **caractérisé en ce que** le dispositif de réglage est relié essentiellement de manière rigide à la plaque (3) dans le sens latéral (8) et médial (11).

3. Dispositif (1) selon la revendication 1 ou 2, **caractérisé en ce que** le dispositif de couplage présente une première surface de butée (12) qui interagit avec une surface correspondante sur le dispositif de réglage et/ou la plaque (3), de sorte que la première surface de butée (12) limite la mobilité du dispositif de couplage par rapport à la plaque (3) dans le sens médial (11).

4. Dispositif (1) selon l'une des revendications 1 à 3, **caractérisé en ce que** le dispositif de couplage comporte latéralement un raccord (22) qui, en interaction avec la plaque (3), forme une butée jusqu'à laquelle le dispositif de couplage peut être inséré médialement dans la plaque (3).

5. Dispositif (1) selon l'une des revendications 1 à 4, **caractérisé en ce que** le dispositif de couplage présente une seconde surface de butée (13), qui se trouve sur le dispositif de réglage en position terminale latérale du dispositif de couplage.

6. Dispositif (1) selon la revendication 5, **caractérisé en ce que** la seconde surface de butée (13) est formée au moins en partie par une seconde surface de butée latérale (13a) qui, en position terminale latérale, se trouve au moins partiellement sur un épaulement ou un col du dispositif de réglage, en particulier sur un col de vis de réglage (23), dans une zone d'une extrémité latérale du dispositif de réglage.

7. Dispositif (1) selon les revendications 5 ou 6, **caractérisé en ce que** la seconde surface de butée (13) est formée au moins partiellement par une seconde surface de butée médiale sur le dispositif de couplage, qui, dans la position terminale latérale, se trouve au moins partiellement sur un filetage du dispositif de réglage.

8. Dispositif (1) selon l'une des revendications 1 à 7, **caractérisé en ce que** le dispositif de réglage est relié à la plaque (3) par un filetage, en prévoyant une sécurité de vissage, en particulier une pièce en plastique, qui augmente le frottement dans le filetage afin d'éviter le desserrage du dispositif de réglage.

9. Dispositif (1) selon l'une des revendications 1 à 8, **caractérisé en ce que** le dispositif de réglage ne peut être vissé dans la plaque (3) que jusqu'à une position prédéfinie, ce qui est réalisé structurellement, notamment avec une clé à vis, de préférence une clé à six pans creux qui, à partir d'une position prédéfinie du dispositif de réglage, n'établit plus de contact avec une contrepartie correspondante, en particulier une vis six pans creuse, dans le dispositif de réglage.

10. Dispositif (1) selon l'une des revendications 1 à 9, **caractérisé en ce que** la première surface de butée (12) et la seconde surface de butée (13) sont disposées sous un angle de butée (δ) l'une par rapport à l'autre, notamment sous un angle différent par rapport à un sens de réglage (16) le long duquel le dispositif de réglage est mobile par rapport à la plaque (3), notamment sous un angle de surface de butée (δ) de 10 degrés à 160 degrés, de préférence de 60 degrés à 120 degrés, de sorte qu'un mouvement du dispositif de réglage le long de la direction de réglage (16) a des effets différents sur la position terminale latérale et la position terminale médiane.

11. Dispositif (1) selon la revendication 10, **caractérisé en ce que** la seconde surface de butée (13) est à peu près perpendiculaire au sens de réglage (16) et que la première surface de butée (12) est approximativement parallèle au sens de réglage (16), en particulier la direction de réglage (16) étant orientée sous un angle (α) par rapport à un axe longitudinal du dispositif de couplage de 0,5 degré à 15 degrés, de préférence de 0,8 degré à 2 degrés.

12. Dispositif (1) selon l'une des revendications 1 à 11, **caractérisé en ce que** le dispositif de réglage est conçu sous forme d'une vis de réglage (9) et le dispositif de couplage sous forme d'une vis à os (5), un axe longitudinal de la vis de réglage (9) étant aligné sur un axe longitudinal de la vis à os (5) sous un angle (α) de 0,5 degré à 15 degrés, en particulier de 0,8 degré à 2 degrés.

13. Dispositif (1) selon l'une des revendications 1 à 12, **caractérisé en ce que** le dispositif de réglage est conçu sous forme d'une vis de réglage (9) disposée dans un filetage (15) dans la plaque (3) et positionnable de manière variable au moyen du filetage (15).

14. Dispositif (1) selon l'une des revendications 1 à 13, **caractérisé en ce que** le dispositif de réglage peut être positionné le long d'un sens de réglage (16), en particulier d'un axe fileté, par rapport à la plaque (3).

15. Dispositif (1) selon la revendication 14, **caractérisé en ce que** le sens de réglage (16) est orienté sous un angle (α) par rapport à un axe longitudinal du dispositif de couplage de 0,5 degré à 10 degrés, en particulier de 0,8 degré à 2 degrés, la direction de réglage (16) et un axe longitudinal du dispositif de couplage se trouvant de préférence sur un plan.
